# FASCICULE DE BREVET EUROPEEN

(11) **EP 0 552 325 B1**
(45) Date de publication et mention de la délivrance du brevet: **26.04.1995**
(21) Numéro de dépôt: 92912517.7
(22) Date de dépôt: 29.06.1992
(51) Int. Cl.: C07C 13/48, C07C 6/04, C07C 11/02

(54) **COMPOSE NAPHTALENIQUE NOUVEAU ET PROCEDE POUR SON OBTENTION**
NEUE NAPHTHALINDERIVATE UND VERFAHREN ZUR HERSTELLUNG
NOVEL NAPHTHALENE COMPOUND AND METHOD OF OBTAINING IT

(30) Priorité: 13.08.1991 CH 2385/91
(43) Date de publication de la demande: 28.07.1993
(73) Titulaire: FIRMENICH SA, 1211 Genève 8 (CH)
(72) Inventeur: LERESCHE, Jean-Paul, CH-1028 Préverenges (CH); MENTHA, Yves, CH-1206 Genève (CH)
(74) Mandataire: Salvadori, Giuseppe
(86) Numéro de dépôt international: CH9200128
(87) Numéro de publication internationale: WO9304023

(56) Documents cités:
- EP-A- 405 427
- EP-A- 0 218 138
- US-A- 4 551 573
- US-A- 4 877 916

## Description

La présente invention a trait au domaine de la synthèse organique. Plus particulièrement, elle concerne un composé naphtalénique nouveau et son utilisation à titre de produit intermédiaire pour la préparation d'un ingrédient parfumant prisé.

### Technique antérieure

La demande de brevet européen publiée sous le n° 405 427-A2 décrit une série de composés naphtaléniques de formule 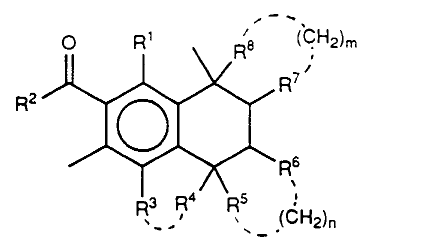
dans laquelle
a) les indices m et n sont identiques et définissent chacun un nombre entier de valeur 0, les symboles R¹ et R² sont identiques et représentent chacun un atome d'hydrogène, ou sont différents et représentent chacun un atome d'hydrogène ou un radical méthyle, les symboles R⁵ et R⁸ représentent chacun un radical méthyle, les symboles R⁶ et R⁷, identiques ou différents, représentent chacun un atome d'hydrogène ou un radical méthyle, et soit le symbole R⁴ représente un radical méthyle et le symbole R³ un atome d'hydrogène ou radical méthyle, soit les symboles R³ et R⁴ représentent chacun un radical méthylène faisant partie d'un cycle tel qu'indiqué par la ligne pointillée, les combinaisons suivantes étant cependant exclues:
   1. R¹ = R² = R³ = R⁶ = R⁷ = H, ou
   2. R¹ = R² = R³ = H et R⁶ ou R⁷ = CH₃, ou
   3. R² = CH₃ et R³ = R⁶ = R⁷ = H, ou
   4. R² = CH₃, R¹ = H R³ = H et R⁶ ou R⁷ = CH₃, ou
   5. R¹ = R³ = CH₃, ou
   6. R³ = R⁴ = CH₂ et R² ou R⁷ = CH₃; ou dans laquelle
b) les indices m et n sont différents et définissent chacun un nombre entier de valeur 0 ou 1, le symbole R² représente un atome d'hydrogène ou un radical méthyle, les symboles R¹ et R³ représentent chacun un atome d'hydrogène, le symbole R⁴ représente un radical méthyle, et soit les symboles R⁵ et R⁶ sent identiques (n=1) et représentent chacun un radical méthylène faisant partie d'un cycle tel qu'indiqué par la ligne pointillée, le symbole R⁷ représentant un atome d'hydrogène et le symbole R⁸ un radical méthyle, soit le symbole R⁵ représente un radical méthyle et le symbole R⁶ un atome d'hydrogène, les symboles R⁷ et R⁸ étant alors identiques (m=1) et représentant chacun un radical méthylène faisant partie d'un cycle tel qu'indiqué par la ligne pointillée.

Il s'agit de composés utiles pour l'industrie de la parfumerie, tout spécialement en vertu de leur caractère musqué, ambré et animal. Parmi ces composés figure un composé d'emploi préférentiel tant pour la nature de son odeur que par sa puissance. Il est apparu en effet à l'expérience que le 5,6,7,8-tétrahydro-3,5,5,6,7,8,8-heptaméthyl-2-naphtalènecarbaldéhyde représente, de par ses qualités, un ingrédient parfumant de choix.

Dans la demande européenne précitée, plusieurs voies de synthèse pour l'obtention de ce composé ont été proposées mais, compte tenu de son intérêt, il n'est pas étonnant de constater que sa préparation a fait l'objet d'une recherche ultérieure, recherche ayant pour but d'en améliorer les coûts de production.

### Exposé de l'invention

C'est dans ce contexte que se situe la présente invention. Elle apporte en effet une solution nouvelle non suggérée dans la référence citée. L'élément critique sur lequel s'articule l'invention est constitué par un intermédiaire utile à la synthèse du 5,6,7,8-tétrahydro-3,5,5,6,7,8,8-heptaméthyl-2-naphtalènecarbaldéhyde. Il s'agit d'un hydrocarbure de formule 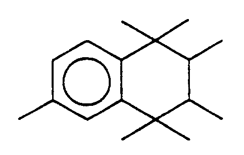
ou 1,1,2,3,4,4,6-heptaméthyl-1,2,3,4-tétrahydronaphtalène (ci-après "HpMT"), lequel peut être transformé en l'aldéhyde désiré par une réaction de formylation selon le schéma suivant: 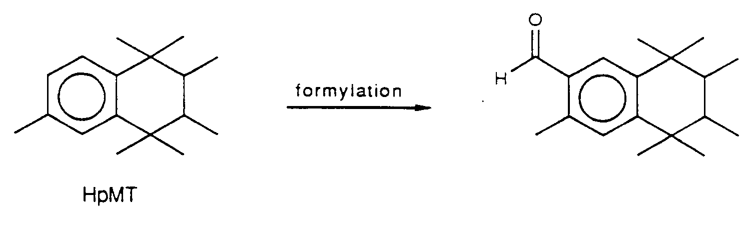
Le HpMT est une entité chimique nouvelle qui peut être obtenue, grâce à un procédé faisant également l'objet de la présente invention, par la réaction entre le p-cymène et une oléfine de formule 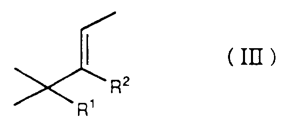
dans laquelle R¹ et R² représentent des substituants différents et chacun définit un atome d'hydrogène ou un radical méthyle. Il s'agit d'une réaction qui peut être effectuée par analogie avec des méthodes connues [voir à cet effet: demande de brevet JP 82 40420 - cf. C.A. 97, 38653 f]. Elle repose sur le principe illustré à l'aide du schéma réactionnel suivant: 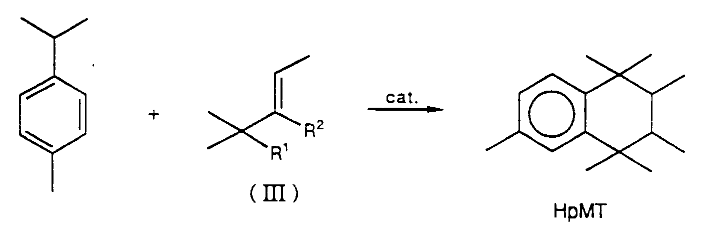
La réaction a lieu en présence de quantités catalytiques de chlorure d'aluminium, auquel on ajoute, le cas échéant, de l'iode [cf. brevet US 4,551,573].

Les proportions de AlCl₃ dans le mélange de réaction peuvent varier dans une gamme de valeurs assez étendue; toutefois, nous avons constaté que des proportions de l'ordre de 3 à 10 mole-% environ, par rapport à la quantité d'oléfine (III), permettaient d'obtenir les rendements les meilleurs. Avec des proportions inférieures à la limite la plus petite indiquée, la réaction procède trop lentement, tandis qu'à des concentrations dépassant la limite supérieure, il y a formation de produits secondaires non désirés, vraisemblablement de structure indanique.

Les proportions relatives des deux réactifs, p-cymène et oléfine (III), ont également une influence critique sur le rendement de la réaction. C'est ainsi que l'emploi d'un excès de p-cymène par rapport à l'oléfine (III) permet l'obtention de HpMT avec des rendements théoriques dépassant 60%. Tout spécialement, un rapport molaire de 2:1, p-cymène/oléfine (III), fournit les rendements les meilleurs.

La réaction peut être conduite soit dans un solvant organique inerte, de préférence chloré comme par exemple le dichlorométhane ou le dichloroéthane, ou encore un hydrocarbure cycloaliphatique comme le cyclohexane, soit en absence de tout solvant par mélange direct des réactifs.

Pour des raisons d'ordre pratique et économique, on effectue la réaction à une température située au voisinage de la température ambiante, de préférence à environ 15° à 30°C.

La présente invention a donc pour objet un procédé pour la préparation de 1,1,2,3,4,4,6-heptaméthyl-1,2,3,4-tétrahydronaphtalène ("HpMT"), lequel procédé est caractérisé en ce qu'on fait réagir du p-cymène avec une oléfine de formule 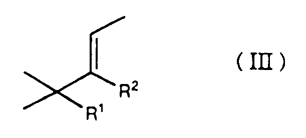
dans laquelle R¹ et R² représentent des substituants différents et chacun définit un atome d'hydrogène ou un radical méthyle, dans un rapport molaire respectif égal ou supérieur à 1:1, en présence d'un système catalytique constitué par du chlorure d'aluminium ou un mélange de chlorure d'aluminium et iode, à une température comprise entre environ 0° et 30°C.

Le HpMT ainsi obtenu se présente sous forme d'un mélange de deux diastéréoisomères, dans un rapport pondéral cis/trans d'environ 1:4,5.

Un autre objet de la présente invention consiste en un procédé pour la préparation de 4,4-diméthyl-2-pentène (ci-après néoheptène), lequel procédé repose sur l'application de la méthode dite de cométathèse [voir par exemple: S. Warwel et al., Chem. Ztg 1983, 107, 115], plus particulièrement de la réaction entre oléfines en présence de catalyseurs appropriés.

Le procédé de préparation du néoheptène selon l'invention est caractérisé en ce qu'on soumet à cométathèse une oléfine de formule 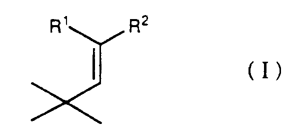
dans laquelle R¹ et R² représentent des substituants identiques désignant chacun un atome d'hydrogène ou un radical méthyle, avec une oléfine de formule 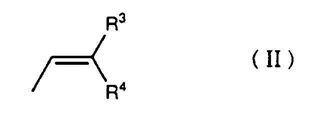
dans laquelle R³ et R⁴, identiques ou différents, servent à désigner chacun un atome d'hydrogène ou un radical méthyle, par contact desdites oléfines, à une température comprise entre environ 30° et 100°C, avec un système catalytique constitué par du Re₂O₇ sur un support solide inerte, ou du WCl₆/(C₂H₅)₂O/Bu₄Sn.

Quoiqu'il était connu que le néohexène [composé (I): R¹=R²=H] subissait des réactions de cométathèse, notamment avec le 5-décène et le 9-octadécène - voir S. Warwel et al., op. cit.-, l'application de ce type de réaction pour l'obtention de néoheptène n'a pas été reportée dans l'art antérieur. Ceci est d'autant plus étonnant que nombreuses ont été à ce jour les synthèses proposées pour l'obtention de cette oléfine.

Comme indiqué plus haut, et en fonction de la nature des différents substituants, la formule (I) peut représenter le néohexène (R¹=R²=H) ou le 2,4,4-triméthyl-2-pentène (R¹=R²=CH₃), tandis que la formule (II) peut représenter le propène (R³=R⁴=H), le 2-méthyl-2-butène (R³=R⁴=CH₃), ou encore le 2-butène (R³=CH₃; R⁴=H ou R³=H; R⁴=CH₃).

La réaction qui caractérise le procédé de l'invention est illustrée par le schéma que voici: 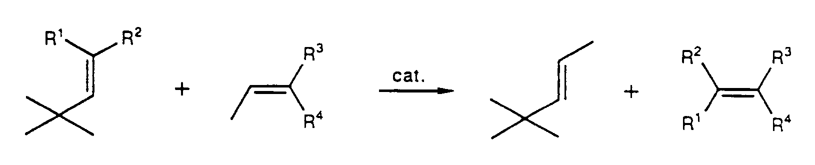
En fonction de la nature des réactifs employés, la réaction peut s'effectuer soit à pression atmosphérique, soit à une pression plus élevée que celle-ci. Avec l'emploi d'oléfines dont le point d'ébullition est inférieur, à pression ordinaire, à la température ambiante, on effectuera la réaction sous pression, de préférence dans un autoclave, tandis qu'avec des réactifs tels par exemple le néohexène et le 2-méthyl-2-butène, la réaction pourra procéder à pression atmosphérique.

A titre de catalyseur, on a mentionné plus haut le Re₂O₇ sur un support solide. L'alumine peut parfaitement convenir en tant que support solide. Nous avons également remarqué qu'il était possible d'augmenter l'activité du catalyseur en ajoutant à celui-ci des dérivés alkylés d'étain, par exemple le tétrabutylétain.

Les proportions respectives de catalyseur et d'oléfines varient dans une gamme étendue de valeurs. Elles sont de préférence comprises entre environ 0,05 et 0,5 mole-%, tandis que la quantité molaire de Re₂O₇ par exemple par rapport à l'adjuvant Bu₄Sn, pouvait être d'environ 1:1 à 1:2. Le contenu de Re₂O₇ dans le mélange avec l'alumine était de l'ordre de 3-10%, de préférence de 5 à 7%, parties en poids.

L'invention est illustrée de manière plus détaillée par les exemples suivants dans lesquels les températures sont indiquées en degrés centigrades et les abréviations ont le sens usuel dans l'art.

### Préparation et (ré)activation du catalyseur Re₂O₇/Al₂O₃

Une solution de 10,0 g d'oxyde de rhénium (VII) (Fluka 83680) dans 600 ml d'eau est placée dans un ballon de 2 l. On y ajoute 170 g d'alumine gamma (Akzo-Ketjen 001-3P) et brasse le tout au moyen d'un Rotavapor pendant 3 h. L'eau est ensuite évaporée sous pression réduite, et l'alumine gamma imprégnée est préséchée à 120° sous 10 mbar pendant 1 h. Après calcination (voir ci-dessous), on obtient 162,4 g de Re₂O₇/Al₂O₃ (6,16%).
L'activation du catalyseur, qui doit être répétée après chaque utilisation, est effectuée de la manière suivante. Le catalyseur est introduit dans un tube de quartz (diam. int.=25 mm) et est chauffé à 550° sous un léger courant d'air pendant 3 h. La température est ensuite augmentée à 600° et le courant d'air est remplacé par un courant d'azote. Après 30 min à cette température, on laisse refroidir, toujours sous courant d'azote. Le catalyseur prêt à l'emploi est stocké sous atmosphère inerte. On a procédé ainsi dans chacune des expériences décrites aux exemples suivants.

### Exemple 1

### Préparation de néoheptène

Dans un ballon à 3 cols équipé d'un thermomètre et d'un réfrigérant à eau surmonté successivement d'une colonne (diam. int.=25 mm, long.=20 cm), remplie d'anneaux de Raschig (diam.=6 mm, long.=6 mm), d'un réfrigérant à double manteau connecté à un cryostat, puis d'un bulleur, on introduit sous contre-courant d'azote 162 g de Re₂O₇/Al₂O₃ (6,16%, soit 20.7 mmole de Re₂O₇).
On y ajoute 870 g (10,35 mole) de néohexène (Phillips Petroleum), 725 g (10,35 mole) de 2-méthyl-2-butène (Shell) et 14,4 g (41,2 mmole) de tétrabutylétain (Fluka 86942). On règle la température du cryostat à -12° et plonge le ballon dans un bain à 45°. L'ébullition, le reflux dans la colonne Raschig et le dégagement de l'isobutylène indiquent que la réaction a démarré. La température du mélange réactionnel, initialement de 27°, s'élève peu à peu. Après 6,8 h de réaction, elle est de 37°. Le mélange réactionnel est refroidi puis filtré, et on obtient 1213 g de produit brut. Une analyse GC montre qu'il contient 22% de 2-méthyl-2-butène, 14% de néohexène et 56% de néoheptène. Le rendement GC brut est de 67%. Calculé par rapport au néohexène consommé, il est de 83%. Une distillation fractionnée permet de récupérer les oléfines de départ non réagies (p.e. 39-41°), puis le néoheptène pur (p.e. 76°).

### Exemple 2

### Préparation de néoheptène

Dans un autoclave Berghof 250 ml, on introduit 9,7 g de Re₂O₇/Al₂O₃ (5,0%) (soit 1,0 mmole Re₂O₇), 34,0 g (0,40 mole) de néohexène et 0,69 g (2,0 mmole) de Bu₄Sn. On ferme l'autoclave et établit une légère dépression, puis on y introduit environ 37 g (0,66 mole, 1,6 éq.) de trans-2-butène (Fluka 19111). On chauffe à 55±5° pendant 2,0 h, avec une agitation modérée. L'autoclave est ensuite refroidi et une trappe froide (eau + glace) est connectée à la vanne de sortie des gaz. Cette vanne est ensuite ouverte pour laisser s'échapper le 2-butène en excès ainsi que les gaz formés (éthylène, propylène). La phase liquide restante est filtrée et on obtient ainsi (y compris le contenu de la trappe) 38,9 g de mélange brut. Une analyse GC montre qu'il contient 15% de 2-butène, 16% de néohexène et 56% de néoheptène. Le rendement GC brut est donc de 55,5%. Basé sur le néohexène consommé, il est de 67%.

### Exemple 3

### Préparation de néoheptène

Dans un autoclave Berghof 250 ml, on introduit 34,7 g (0,41 mole) de néohexène, 1,8 g (5,2 mmole) de Bu₄Sn et 0,20 g (2,7 mmole) de diéthyléther. On enclenche l'agitation (barreau magnétique) et ajoute sous contre-courant d'azote 1,0 g (2,5 mmole) de WCl₆ (Fluka 95400). On ferme l'autoclave et établit une légère dépression, puis on y introduit environ 34,7 g (0,62 mole, 1,5 éq.) de trans-2-butène. On chauffe à 100±10° pendant 90 min, avec une agitation vigoureuse. L'autoclave est ensuite refroidi et la vanne de sortie des gaz est ouverte pour laisser s'échapper le 2-butène en excès ainsi que les gaz formés (éthylène, propylène). On ajoute ensuite à la phase liquide restante 30 ml d'une solution aqueuse de NaOH à 10%. On sépare les phases, lave la phase organique avec encore 30 ml de NaOH aq. 10%, 30 ml de HCl aq. 5%, 30 ml d'eau et enfin 30 ml de NaCl aq. sat. On sèche sur sulfate de sodium, filtre et obtient ainsi 38,3 g de mélange brut. Une analyse GC montre qu'il contient 12% de 2-butène, 20% de néohexène et 55% de néoheptène. Le rendement GC brut est donc de 52%. Basé sur le néohexène consommé, il est de 67%.

### Exemple 4

### Préparation de HpMT

Dans un ballon à 3 cols de 100 ml équipé d'un thermomètre, d'un réfrigérant surmonté d'un bulleur d'azote, d'une ampoule d'introduction et d'un agitateur magnétique, on introduit 25 ml de dichlorométhane sec et 0,80 g (0,006 mole) de chlorure d'aluminium finement divisé (Fluka 06220). On refroidit à 15° et ajoute en 40 min un mélange de 32,2 g (0,24 mole) de p-cymène et 11,8 g (0,12 mole) de néoheptène tout en maintenant la température à 15±2°. Après la fin de l'addition, on laisse revenir à température ambiante et poursuit l'agitation encore 3 h. Le mélange réactionnel est refroidi à environ 5° avant d'y ajouter 50 ml d'eau froide. Après séparation des phases, la phase aqueuse est extraite à l'éther diéthylique, puis les phases organiques réunies sont lavées avec 50 ml d'eau et 50 ml de NaCl aq. sat., puis séchées sur sulfate de sodium et concentrées au Rotavapor. On obtient ainsi 35,2 g de produit brut qui contient (GC) 21,7% de HpMT et 63,6% de p-cymène. Une portion de 3,0 g est distillée au four à boules et fournit une première fraction de 1,85 g de p-cymène (95% GC) et une seconde fraction de 1,07 g contenant 11,6% de cis- et 55,1% de trans-HpMT. Le taux de résidu est donc inférieur à 3% et le rendement de la réaction, basé sur le néoheptène consommé, est de 60,5%. Un échantillon de trans-1,1,2,3,4,4,6-heptaméthyl-1,2,3,4-tétrahydronaphtalène pur, p.f.=65-66°, a été obtenu par cristallisation dans un mélange AcOEt/EtOH (1:1).
- MS:: 230(M⁺,12%), 215(38), 173(56), 159(100), 141(14), 131(43), 57(84), 41(17)
- ¹H-RMN (360MHz,CDCl₃):: 0,96(d,J=5,49Hz,6H); 1,08(s,3H); 1,09(s,3H); 1,29(s,3H); 1,31(s,3H); 1,58(m,2H); 2,30(s,3H); 6,96(dd,J=7,93 et 1,83Hz,1H); 7,15(d,J=1,83Hz,1H); 7,25(d,J=7,93,1H) δ ppm
- ¹³C-RMN (90,54MHz,CDCl₃):: 13,79(q,2CH₃), 21,09(q), 25,59(q), 25,62(q), 29,52(q), 29,61(q), 37,55(s), 37,79(s), 39,41(d,2CH), 126,51(d), 127,09(d), 127,60(d), 134,53(s), 142,68(s), 145,46(s)
- IR(KBr):: 2973 (intense), 1662(i), 1458(moyenne), 1208(m), 1168(m), 824(m) cm⁻¹
D'autres exemples spécifiques de préparation de HpMT sont résumés dans le tableau suivant ^{a)}:
a) réactions effectuées avec 0,12 mole de néoheptène (4,4-diméthyl-2-pentène)

| Exp. | p-cym (mole) | AlCl₃ (mole) | Solvant | Quantité de solvant | T (°C) | Rdt (%) |
|---|---|---|---|---|---|---|
| 1) | 0,135 | 0,006 | CH₂Cl₂ | 25 ml | 15→25 | 54 |
| 2) | 0,135 | 0,006 | CH₂Cl₂ | 0,06 mole | 15→25 | 41 |
| 3) | 0,135 | 0,006 | (ClCH₂)₂ | 25 ml | 15→25 | 56 |
| 4) | 0,135 | 0,006 | (ClCH₂)₂ | 0,06 mole | 15→25 | 40 |
| 5) | 0,135 | 0,006 | cyclohexane | 25 ml | 15→25 | 43 |
| 6) | 0,135 | 0,006 | - | - | 15→25 | 39 |
| 7) | 0,135 | 0,030 | CH₂Cl₂ | 25 ml | 15→25 | <5 |
| 8) | 0,135 | 0,0012 | CH₂Cl₂ | 25 ml | 15→25 | 19 |
| 9) | 0,060 | 0,006 | CH₂Cl₂ | 25 ml | 15→25 | 47 |
| 10) | 0,135 | 0,006 | CH₂Cl₂ | 25 ml | 0 | 50 |
| 11) | 0,135 | 0,006 | CH₂Cl₂ | 25 ml | 25 | 54 |

Les résultats obtenus dans les exemples précédents ainsi que dans d'autres exemples spécifiques de préparation du néoheptène sont résumés dans le tableau suivant :

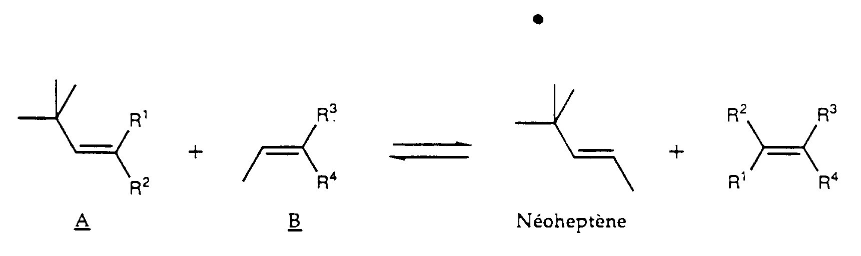

| Exp. | R¹ | R² | R³ | R⁴ | Cat. | Cat/A (poids) | B/A | Temps (h) | Temp. (°C) | Conv. (%) | Rdt (%) |
|---|---|---|---|---|---|---|---|---|---|---|---|
| 1) | H | H | H | H | I | 0,2 | 1,0 | 10 | 40 | 25,7 | 26 |
| 2) | CH₃ | CH₃ | CH₃ | H | I | 0,4 | 4,8 | 5 | 40 | 93,3 | 59 |
| 3) | CH₃ | CH₃ | CH₃ | H | I | 0,8 | 2,4 | 6 | 40 | 99,4 | 59 |
| 4) | CH₃ | CH₃ | CH₃ | H | II | 0,8 | 7,6 | 3 | 40 | 99,6 | 87 |
| 5) | H | H | CH₃ | CH₃ | III | 0,2 | 1,0 | 7 | 27-37 | 81 | 67 |
| 6) | H | H | CH₃ | CH₃ | IV | 0,15 | 1,0 | 3 | 40 | | 0 |
| 7) | H | H | CH₃ | H | III | 0,3 | 1,6 | 2 | 55 | 82 | 55 |
| 8) | H | H | CH₃ | H | IV | 0,08 | 1,5 | 1,5 | 100 | 77 | 52 |
| 9) | CH₃ | CH₃ | CH₃ | H | IV | 0,10 | 3,7 | 3 | 80 | | 0 |
| I = Re₂O₇/Al₂O₃ II = Re₂O₇/Al₂O₃ + Me₄Sn III = 5-7% Re₂O₇/Al₂O₃ + Bu₄Sn; (Re₂O₇: Bu₄Sn: oléfines = 1:2:1000) IV = WCl₆-Et₂O-Bu₄Sn; (WCl₆: Et₂O: Bu₄Sn: oléfines = 1:1:2:500) | | | | | | | | | | | |

## Revendications

1. 1,1,2,3,4,4,6-Heptaméthyl-1,2,3,4-tétrahydronaphtalène.

2. Procédé pour la préparation de 1,1,2,3,4,4,6-heptaméthyl-1,2,3,4-tétrahydronaphtalène caractérisé en ce qu'on fait réagir du p-cymène avec une oléfine de formule 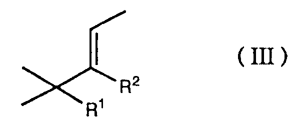 dans laquelle R¹ et R² représentent des substituants différents et chacun définit un atome d'hydrogène ou un radical méthyle, dans un rapport molaire respectif égal ou supérieur à 1:1, en présence d'un système catalytique constitué par du chlorure d'aluminium ou un mélange de chlorure d'aluminium et iode, à une température comprise entre 0° et 30°C.

3. Procédé suivant la revendication 2, caractérisé en ce que la réaction s'effectue dans un solvant organique inerte.

4. Procédé suivant la revendication 3, caractérisé en ce que le solvant organique inerte est le dichlorométhane ou le dichloroéthane.

5. Procédé suivant la revendication 3, caractérisé en ce que le solvant organique inerte est le cyclohexane.

6. Procédé suivant la revendication 1, caractérisé en ce que le rapport molaire respectif de p-cymène et de l'oléfine (III) est d'environ 2:1.

7. Procédé selon l'une quelconque des revendications 2 à 6, caractérisé en ce que l'oléfine (III) est le 4,4-diméthyl-2-pentène.

8. Procédé pour la préparation de 4,4-diméthyl-2-pentène caractérisé en ce qu'on soumet à cométathèse une oléfine de formule 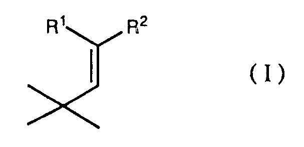 dans laquelle R¹ et R² représentent des substituants identiques désignant chacun un atome d'hydrogène ou un radical méthyle, avec une oléfine de formule 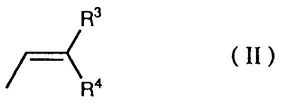 dans laquelle R³ et R⁴, identiques ou différents, servent à désigner chacun un atome d'hydrogène ou un radical méthyle, par contact desdites oléfines, à une température comprise entre environ 30° et 100°C, avec un système catalytique constitué par du Re₂O₇ sur un support solide inerte, ou du WCl₆/(C₂H₅)₂O/Bu₄Sn.

9. Procédé suivant la revendication 8, caractérisé en ce que la réaction s'effectue à pression atmosphérique ou à une pression plus élevée que celle-ci.

10. Procédé suivant la revendication 8, caractérisé en ce qu'on soumet à cométathèse le néohexène avec le propène.

11. Procédé suivant la revendication 8, caractérisé en ce qu'on soumet à cométathèse le 2,4,4-triméthyl-2-pentène avec le 2-butène.

12. Procédé suivant la revendication 8, caractérisé en ce qu'on soumet à cométathèse le néohexène avec le 2-méthyl-2-butène.

13. Procédé suivant la revendication 8, caractérisé en ce qu'on soumet à cométathèse le néohexène avec le 2-butène.

## Claims

1. 1,1,2,3,4,4,6-Heptamethyl-1,2,3,4-tetrahydronaphthalene.

2. Process for the preparation of 1,1,2,3,4,4,6-heptamethyl-1,2,3,4-tetrahydronaphthalene characterized in that p-cymene is reacted with an olefin of formula 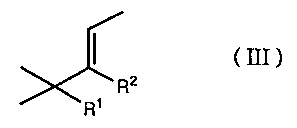 wherein R¹ et R² represent different substituents and each defines a hydrogen atom or a methyl radical, in a respective molar ratio equal to or above 1:1, in the presence of a catalytic system consisting of aluminium chloride or of a mixture of aluminium chloride and iodine, at a temperature comprised between 0° and 30°C.

3. Process according to claim 2, characterized in that the reaction is effected in an inert organic solvent.

4. Process according to claim 3, characterized in that the inert organic solvent is dichloromethane or dichloroethane.

5. Process according to claim 3, characterized in that the inert organic solvent is cyclohexane.

6. Process according to claim 1, characterized in that the respective molar ratio of p-cymene and olefin (III) is about 2:1.

7. Process according to anyone of claims 2 to 6, characterized in that the olefin (III) is 4,4-dimethyl-2-pentene.

8. Process for the preparation of 4,4-dimethyl-2-pentene, characterized in that an olefin of formula 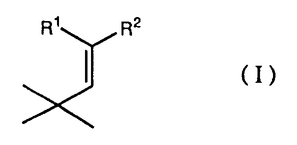 wherein R¹ and R² represent identical substituents designating each a hydrogen atom or a methyl radical, is subjected to co-metathesis with an olefin of formula 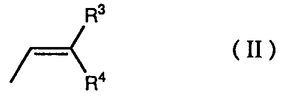 wherein R³ and R⁴, identical or different, designate each a hydrogen atom or a methyl radical, via contact of said olefins, at a temperature comprised between around 30° and 100°C, with a catalytic system consisting of Re₂O₇ on an inert solid carrier, or of WCl₆/(C₂H₅)₂O/Bu₄Sn.

9. Process according to claim 8, characterized in that the reaction is effected at atmospheric pressure or at a pressure above the latter.

10. Process according to claim 8, characterized in that neohexene is subjected to co-metathesis with propene.

11. Process according to claim 8, characterized in that 2,4,4-trimethyl-2-pentene is subjected to co-metathesis with 2-butene.

12. Process according to claim 8, characterized in that neohexene is subjected to co-metathesis with 2-methyl-2-butene.

13. Process according to claim 8, characterized in that neohexene is subjected to co-metathesis with 2-butene.

## Patentansprüche

1. 1,1,2,3,4,4,6-Heptamethyl-1,2,3,4-tetrahydronaphthalin.

2. Verfahren zur Herstellung von 1,1,2,3,4,4,6-Heptamethyl-1,2,3,4-tetrahydronaphthalin, dadurch gekennzeichnet, dass man p-Cymen mit einem Olefin der Formel 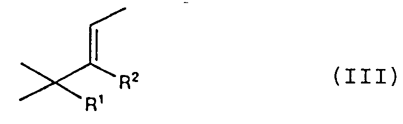 worin R¹ und R² verschiedene Substituenten bedeuten und jeder ein Wasserstoffatom oder einen Methylrest definiert, in einem beiderseitigen molaren Verhältnis von gleich oder mehr als 1:1 in Anwesenheit eines Katalysatorsystems, das aus Aluminiumchlorid oder einem Gemisch von Aluminiumchlorid und Jod gebildet wird, bei einer Temperatur zwischen 0° und 30°C umsetzt.

3. Verfahren gemäss Patentanspruch 2, dadurch gekennzeichnet, dass die Reaktion in einem inerten organischen Lösungsmittel erfolgt.

4. Verfahren gemäss Patentanspruch 3, dadurch gekennzeichnet, dass das inerte organische Lösungsmittel Dichlormethan oder Dichlorethan ist.

5. Verfahren gemäss Patentanspruch 3, dadurch gekennzeichnet, dass das inerte organische Lösungsmittel Cyclohexan ist.

6. Verfahren gemäss Patentanspruch 1, dadurch gekennzeichnet, dass das beiderseitige molare Verhältnis des p-Cymens und des Olefins (III) ungefähr 2:1 beträgt.

7. Verfahren gemäss irgendeinem der Patentansprüche 2 bis 6, dadurch gekennzeichnet, dass das Olefin (III) 4,4-Dimethyl-2-penten ist.

8. Verfahren zur Herstellung von 4,4-Dimethyl-2-penten, dadurch gekennzeichnet, dass dass man ein Olefin der Formel 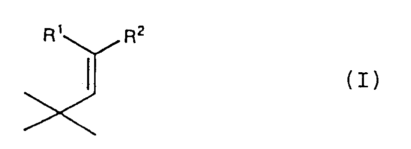 worin R¹ und R² gleiche Substituenten bedeuten, wobei jeder für ein Wasserstoffatom oder einen Methylrest steht, mit einem Olefin der Formel 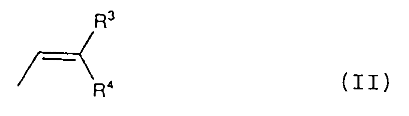 worin R³ und R⁴ gleich oder verschieden sind und jedes dazu dient, ein Wasserstoffatom oder einen Methylrest zu bezeichnen, durch Kontakt dieser Olefine bei einer Temperatur zwischen ungefähr 30° und 100°C, mit einem katalytischen System, das gebildet wird aus Re₂O₇ auf einem inerten festen Träger oder WCl₆/(C₂H₅)₂O/Bu₄Sn, einer Cometathese unterwirft.

9. Verfahren gemäss Patentanspruch 8, dadurch gekennzeichnet, dass die Reaktion bei Atmosphärendruck oder bei einem höheren Druck als diesem erfolgt.

10. Verfahren gemäss Patentanspruch 8, dadurch gekennzeichnet, dass man Neohexen mit Propen einer Cometathese unterwirft.

11. Verfahren gemäss Patentanspruch 8, dadurch gekennzeichnet, dass man 2,4,4-Trimethyl-2-penten mit 2-Buten einer Cometathese unterwirft.

12. Verfahren gemäss Patentanspruch 8, dadurch gekennzeichnet, dass man Neohexen mit 2-Methyl-2-buten einer Cometathese unterwirft.

13. Verfahren gemäss Patentanspruch 8, dadurch gekennzeichnet, dass man Neohexen mit 2-Buten einer Cometathese unterwirft.
